(19) Europäisches Patentamt
European Patent Office
Office européen des brevets

(11) Veröffentlichungsnummer: **0 169 299**
**B1**

(12) **EUROPÄISCHE PATENTSCHRIFT**

(45) Veröffentlichungstag der Patentschrift: **25.04.90**

(51) Int. Cl.$^5$: **C 07 C 229/16,** G 01 R 33/56

(21) Anmeldenummer: **85102713.6**

(22) Anmeldetag: **19.07.82**

(60) Veröffentlichungsnummer der früheren Anmeldung nach Art. 76 EPÜ: **0 071 564**

# (54) Verwendung von paramagnetischen Komplexsalzen zur Herstellung von Mitteln für die NMR-Diagnostik.

(30) Priorität: **24.07.81 DE 3129906**

(43) Veröffentlichungstag der Anmeldung: **29.01.86 Patentblatt 86/05**

(45) Bekanntmachung des Hinweises auf die Patenterteilung: **25.04.90 Patentblatt 90/17**

(84) Benannte Vertragsstaaten: **AT BE CH DE FR GB IT LI LU NL SE**

(56) Entgegenhaltungen:
**FR-A-2 159 411**

**JOURNAL OF THE AMERICAN CHEMICAL SOCIETY, Band 99, Nr. 6, 16. März 1977, Seiten 1762-1765, Washington, D.C., US; G.A. ELGAVISH et al.: "Aqueous lanthanide shift reagents. 3. Interaction of the ethylenediaminetetraacetate chelates with substituted ammonium cations"**

(73) Patentinhaber: **SCHERING AKTIENGESELLSCHAFT Berlin und Bergkamen
Müllerstrasse 170/178 Postfach 65 03 11
D-1000 Berlin 65 (DE)**

(72) Erfinder: **Gries, Heinz, Dr.
Helmstedter Strasse 19
D-1000 Berlin 31 (DE)**
Erfinder: **Rosenberg, Douwe, Dr.
Pariser Strasse 9
D-1000 Berlin 15 (DE)**
Erfinder: **Weinmann, Hanns-Joachim, Dr.
bei Fischer Niebuhrstrasse 61
D-1000 Berlin 12 (DE)**

**EP 0 169 299 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft die Verwendung von physiologisch verträglichen paramagnetischen Komplexsalzen zur Herstellung von Mitteln für die NMR-Diagnostik. Diese Komplexsalze bestehen aus Aminopolycarbonsäuren der Formeln I bis IV

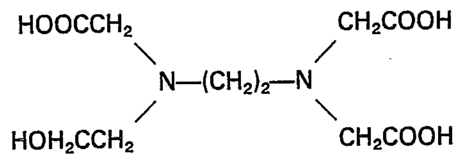

(I)

N-Hydroxyäthyl-N,N',N'-äthylendiamin-triessigsäure (HEDTA),

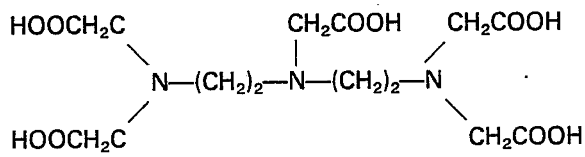

(II)

N,N,N',N'',N''-Diäthylentriamin-pentaessigsäure (DTPA),

$HOH_2C{-}CH_2N(CH_2COOH)_2$ (III)

N-Hydroxyäthyliminodiessigsäure,

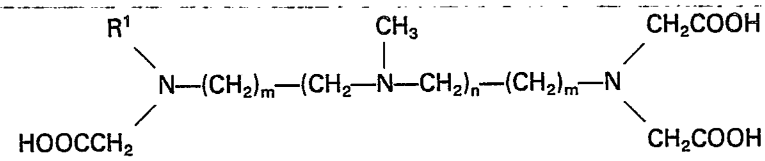

(IV),

worin
m die Zahlen 1 bis 4,
n die Zahlen 0 bis 2,
$R^1$ einen gesättigten oder ungesättigten Kohlenwasserstoffrest mit 4 bis 12 Kohlenwasserstoffatomen oder die Gruppe $-CH_2-COOH$ darstellt, und
den Ionen der Lanthaniden-Elemente der Ordnungszahlen 57 bis 70 oder
den Ionen der Übergangsmetalle der Ordnungszahlen 21 bis 29, 42 und 44
und gegebenenfalls einer anorganischen Base.

Bevorzugte Komplexbildner sind N,N,N',N' - Äthylendiamin - tetraessigsäure (EDTA) und N,N,N',N'',N'' - Diäthylentriamin - pentaessigsäure (DTPA).

Diese Komplexe sind bischer nicht als NMR-Diagnostika verwendet worden. So sind zum Beispiel Praseodym-, Neodym-, Europium-EDTA- bzw. Praseodym-, Gadolinium-, Ytterbium-EDTA-Ammonium- und Pyridinium-Komplexe, wie in J. Am. Chem. Soc. *98*, 3726 (1976) bzw. in J. Am. Chem. Soc. *99*, 1762 (1977) beschrieben, nur für physikalische Untersuchungen eingesetzt worden.

Anwendung in der Humanmedizin fanden bisher nur die Kupfer, Kobalt- und Eisen-Metallsalze der EDTA: In der Patentschrift FR—A—988 M (Laboratoires Gerda) werden des Kupfer-(II)-EDTA und dessen Dinatriumsalz als Mittel zur Behandlung des Rheumatismus beschrieben; die Patentschrift FR—A—484 M (Fabriques de produits chimiques Billault) befaßt sich mit dem Eisen(II)-Salz des Eisen(III)-Komplexes der EDTA als Mittel gegen Anämie; in der Patentschrift FR—A—1 111 504 (Cassella Farbwerke Mainkur) werden das Dinatrium- und Dikalium- salz des Kobalt-Komplexes der EDTA als Therapeutikum zur Blutbildung beschrieben. Für die NMR-Diagnostik sind bisher weder diese noch andere erfindungsgemäße Komplexsalze mit den Verbindungen der Formeln I bis IV verwendet worden.

Wenn erforderlich, ist es auch moglich, die erfindungsgemäßen Komplexverbindungen an Biomoleküle zu binden, um so die Komplexverbindungen an bestimmte Stellen des lebenden Körpers transportieren zu lassen.

Als Biomoleküle werden beispielsweise verwendet Immunglobuline, Hormone wie Insulin, Glucagen, Prostaglandine, Steroidhormone, Proteine, Peptide, Aminozucker, Lipide.

Die Kopplung der paramagnetischen Komplexsalze an die gewünschten Biomoleküle erfolgt nach an sich bekannten Methoden beispielsweise durch Reaktion der nucleophilen Gruppe eines Biomoleküles wie der Amino-, Phenol-, Sulfhydryl- oder Imidazolgruppe mit einem aktivierten Derivat der Komplexverbindung.

Als aktiviertes Derivat kommen beispielsweise Säurechloride, gemischte Anhydride (herstellbar aus

dem Carboxylderivat der Komplexverbindung mit Chlorkohlensäureester), aktivierte Ester, Nitrene oder Isothiocyanate infrage.

Ebenso ist es möglich, ein aktiviertes Derivat des Biomoleküles mit einem nucleophilen Derivat der Komplexverbindung zur Reaktion zu bringen.

Als Basen werden zur Salzbildung verwendet anorganische Basen, wie zum Beispiel Natriumhydroxid.

Die Herstellung der neuen Mittel erfolgt in an sich bekannter Weise, indem man das paramagnetische Komplexsalz in Wasser oder physiologischer Salzlösung löst, gegebenenfalls unter Zusatz von in den Galenik üblichen Zusätzen wie beispielsweise physiologisch verträglichen Pufferlösungen (z.B. Natriumdihydrogenphosphatlösung) und die Lösung sterilisiert. Die wäßrigen Lösungen können oral, neural und insbesondere intravasal appliziert werden. Sind besonders für die orale Verabreichung Suspensionen der paramagnetischen Komplexsalze in Wasser oder physiologischer Salzlösung erwünscht, wird das paramagnetische Komplexsalz mit einem oder mehreren in der Galenik üblichen Hilfsstoffen und/oder Tensiden und/oder Aromastoffen zur Geschmackskorrektur gemischt und vor der oralen Anwendung in Wasser oder physiologischer Salzlösung suspendiert. Man verwendet hierbei vorzugsweise 3 bis 10 g paramagnetisches Komplexsalz und 2 bis 8 g eines oder mehrerer Hilfsstoffe wie beispielsweise Saccharose, hochdisperses Siliziumdioxid, Polyoxyäthylenpolyoxypropylen - Polymere, Stärke, Magnesiumstearat, Natriumlaurylsulfat, Talkum, Lactose, Carboxymethylcellulose - Natrium.

Zur NMR-Diagnostik beim Menschen werden wäßrige Lösungen oder Suspensionen verwendet, die 5 bis 250 mMol/l, vorzugsweise 50 bis 200 mMol/l, eines paramagnetischen Komplexsalzes enthalten. Die wäßrigen Lösungen liegen im pH-Bereich zwischen etwa 6,5 und 8,0, vorzugsweise zwischen 6,5 und 7,5.

Durch die Komplexbildung gemäß vorliegender Erfindung werden die paramagnetischen Salze entgiftet, und es wird außerdem erreicht, daß die Salze auch im physiologischen pH-Bereich in Wasser beständig und gut löslich sind.

Besonders geeignet erscheinen die neuen Mittel in Form der Komplexsalzlösungen zur besseren Abgrenzung bzw. Lokalisation von Läsionen der Bauchspeicheldrüse und der Leber sowie von Tumoren und Blutungen im cranialen Bereich. Zur Diagnose des zu untersuchenden Areals wird beispielsweise eine wäßrige, zum Blut isotonische Lösung paramagnetischer Komplexsalze in einer Dosis von 1 bis 100 µMol/kg intravenös appliziert. Bei einer Konzentration des Komplexsalzes von 50 bis 200 mMol/l werden für eine Untersuchung am Menschen etwa 1 bis 50 ml Lösung benötigt. Die Aufnahme der interessierenden Schicht erfolgt etwa 15 bis 60 Minuten nach der intravenösen Applikation der wäßrigen Lösung des paramagnetischen Komplexsalzes.

Die in der medizinischen Praxis üblichen physikalischen Diagnoseverfahren, die ohne oder nur mit geringfügigen operativen Eingriffen ausgeführt werden können, sind z.B. die Durchstrahlung des Körpers mit Röntgenlicht, die Szintigraphie und die Sonographie. Alle diese Methoden sind entweder mit gesundheitlichen Risiken behaftet oder der Anwendungsbereich ist eingeschränkt. So ist der Patient bei den Röntgentechniken und bei der Szintigraphie der ionisierenden Strahlung ausgesetzt, so daß diese Methoden nicht beliebig oft oder gar nicht bei Risikogruppen, wie beispielsweise bei Säuglingen und Schwangeren angewendet werden können.

Die Sonographie besitzt zwar die genannten Nachteile nicht, dafür ist aber ihr Anwendungsbereich sehr beschränkt, insbesondere im cranialen Bereich.

Da es trotz großem Forschungsaufwand bisher noch nicht gelungen ist, die geschilderten Nachteile vollständig zu beseitigen, sucht man nach bildgebenden Verfahren, die diese Nachteile nicht besitzen, aber einen vergleichbaren Informationsgewinn für die Diagnose liefern.

Eines dieser bildgebenden Verfahren ist die Kernspin-Tomographie (Spin-Imaging, Zeugmatographie), die auf dem physikalischen Effekt der sogenannten Kernspinresonanz (Nuclear Magnetic Resonance, NMR) beruht. Dieses Diagnoseverfahren ermöglicht es, Schnittbilder des lebenden Körpers und Einblick in Stoffwechselvorgänge zu erhalten ohne Anwendung ionisierender Strahlen. Den Effekt der Kernresonanz zeigen Atomkerne, die—wie Wasserstoff, der im biologischen Gewebe hauptsächlich als Wasser vorhanden ist—ein magnetisches Moment besitzen und sich dadurch in einem starken äußeren Magnetfeld ausrichten. Durch einen Hochfrequenz-Impuls (Resonanzfrequenz) werden sie aus ihrer Gleichgewichtslage gebracht, in die sie mit einer charakteristischen Geschwindigkeit wieder zurückkehren. Die Dauer der Rückkehr in den Gleichgewichtszustand, die sogenannte Relaxationszeit, gibt Auskunft über den Ordnungsgrad der Atome und über ihre Wechselwirkung mit ihrer Umgebung.

Die bildliche Darstellung, die man durch die Messung der Protonendichte bzw. der Relaxationszeiten erhält, ist von hohem diagnostischen Wert und gibt Auskunft über den Wassergehalt und über den Zustand des untersuchten Gewebes. So zeigt Tumorgewebe beispielsweise längers Relaxationszeiten als gesundes Vergleichsgewebe. (A. Ganssen u.a. Computertomographie 1, [1981] S. 2—10; Georg Thieme Verlag, Stuttgart, New York).

Man hat nun festgestellt, daß paramagnetische Ionen, wie beispielsweise $Mn^{2+}$ (Mangan) oder $Cu^{2+}$ (Kupfer) die Relaxationszeiten beeinflussen und damit den Informationsgehält erhöhen.

Die bischer hierfür am Versuchstier verwendeten Schwermetallsalzlösungen sind jedoch für die intravenöse Applikation am Menschen wegen ihrer hohen Toxizität ungeeignet. Man sucht daher nach paramagnetischen Substanzen, die gut vertragen werden und die Bildgebung günstig beeinflussen. Letzteres kann beispielsweise dadurch erfolgen, daß möglichst organspezifisch die Spin-Gitter -Relaxationszeit $T_1$ stark herabgesetzt wird, während gleichzeitig die Spin-Spin-Relaxationszeit $T_2$

weitgehend konstant gehalten wird. Es wurde nun gefunden, daß die gewünschte Entgiftung der sonst toxischen Metallsalze durch eine Komplexierung erfolgen kann, ohne daß die paramagnetischen Eigenschaften ungünstig beeinflußt werden. Letzteres ist überraschend, da hierdurch bekanntlich die Verteilung der d- bzw. f-Elektronen über die d- bzw. f-Orbitale verändert wird.

Die Herstellung der paramagnetischen Komplexsalze erfolgt nach dem Fachmann bekannten Verfahren indem man das paramagnetische Metallsalz der Lanthaniden-Elemente der Ordnungszahlen 57 bis 70 oder der Übergangsmetalle der Ordnungszahlen 21 bis 29, 42 und 44 in Wasser und/oder Alkohol löst und mit der Lösung der äquivalenten Menge des Komplexbildners in Wasser und/oder Alkohol versetzt und rührt, falls erforderlich unter Erhitzen auf 50°C bis 120°C bis die Umsetzung beendet ist. Wird Alkohol als Lösungsmittel verwendet, so wird Methanol oder Äthanol benutzt.

Wenn der gebildete Komplex im verwendeten Lösungsmittel unlöslich ist, kristallisiert er und kann abfiltriert werden. Ist er löslich, kann er durch Eindampfen der Lösung zur Trockne isoliert werden.

Die nachfolgenden Beispiele sollen die Erfindung näher erläutern.

Beispiel 1

a) Herstellung des Mangan-II-Komplexes der Äthylendiamintetraessigsäure:

Die Suspension von 6,17 g Mangan-II-carbonat in 500 ml Wasser wird mit 14,6 g Äthylendiamintetraessigsäure versetzt und unter Rühren auf dem Dampfbad erwärmt, wobei eine Gasentwicklung auftritt. Die anfänglich rosa Farbe verschwindet nach ca. 20 Minuten und es geht alles bis auf einen kleinen Rest in Lösung. Nach einer Stunde rühren bei 110°C wird vom Ungelösten abfiltriert und das Filtrat abgekühlt. Nach 15 Stunden Stehen wird das Kristallisat abgesaugt und getrocknet:

$K_1$=14,1 g (Molgewicht 345,17)

Fp.: 256°/258—259°C.

b) Herstellung einer Lösung vom Di-natriumsalz des Mangan(II)-Komplexes der Äthylendiamintetraessigsäure:

5,55 g (15 mMol) des Mangan(II)-Komplexes der Äthylendiamin-tetraessigsäure (Wassergehalt: 6,9%) werden in 80 ml Wasser p.i. unter Zusatz verdünnter Natronlauge bei pH 7,5 gelöst. Anschließend wird die Lösung mit Wasser p.i. auf 170 ml aufgefüllt, in Ampullen filtriert und hitzesterilisiert.

Beispiel 2

Herstellung des Gadolinium-III-Komplexes der Diäthylentriamin-pentaessigsäure:

Die Suspension von 435 g Gadoliniumoxid ($Gd_2O_3$) und 944 g Diäthylentriamin-pentaessigsäure in 12 Liter Wasser wird unter Rühren auf 90°C bis 100°C erwärmt und 48 Stunden bei dieser Temperatur gerührt. Dann filtriert man vom Ungelösten ab und dampft das Filtrat zur Trockne. Der amorphe Rückstand wird pulverisiert. Ausbeute 144 g; (Molargewicht 547,58).

Fp.: schmilzt ab 235° und bleibt bis 320°C unzersetzt.

Ist (sind) in der erhaltenen paramagnetischen Komplexverbindung noch eine oder mehrere saure Gruppe(n) enthalten, so kann gewünschtenfalls die erhaltene Komplexverbindung anschließend in Wasser gelöst oder suspendiert und mit der gewünschten anorganischen Base versetzt werden, bis der Neutralpunkt erreicht ist. Nach Filtration von ungelösten Anteilen wird die Lösung eingedampft und als Rückstand das gewünschte Komplexsalz erhalten.

**Patentansprüche**

1. Verwendung von mindestens einem physiologisch verträglichen paramagnetischen Komplexsalz aus Aminopolycarbonsäuren der Formeln I bis IV

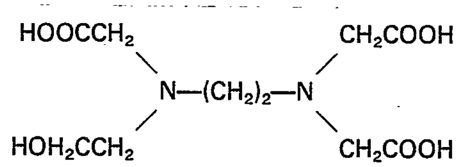

(I)

N-Hydroxyäthyl-N,N',N'-äthylendiamin-triessigsäure (HEDTA),

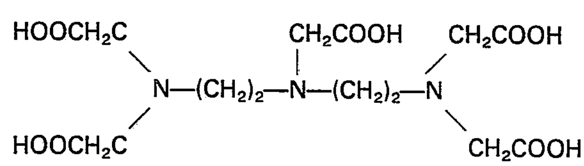

(II)

N,N,N',N'',N''-Diäthylentriamin-pentaessigsäure (DTPA),

$$HOH_2C—CH_2N(CH_2COOH)_2 \quad (III)$$

N-Hydroxyäthyliminodiessigsäure,

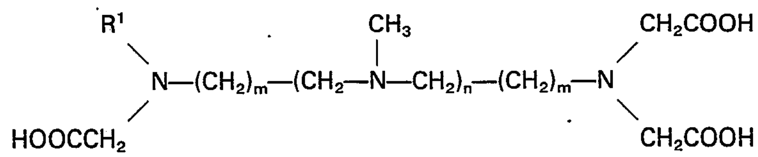

(IV),

worin

m die Zahlen 1 bis 4,

n die Zahlen 0 bis 2,

$R^1$ einen gesättigten oder ungesättigten Kohlenwasserstoffrest mit 4 bis 12 Kohlenwasserstoffatomen oder die Gruppe $-CH_2-COOH$ darstellt, und

den Ionen der Lanthaniden-Elemente der Ordnungszahlen 57 bis 70 oder

den Ionen der Übergangsmetalle der Ordnungszahlen 21 bis 29, 42 und 44

und gegebenenfalls

einer anorganischen Base

zur Herstellung von Mitteln für die NMR-Diagnostik.

2. Verwendung von mindestens einem physiologisch verträglichen paramagnetischen Komplexsalz nach Anspruch 1 zur Herstellung von Mitteln für die NMR-Diagnostik, wobei als anorganische Base Natriumhydroxid dient.

3. Verwendung von mindestens einem physiologisch verträglichen paramagnetischen Komplexsalz nach Anspruch 1 zur Herstellung von Mitteln für die NMR-Diagnostik, wobei als Aminopolycarbonsäure N,N,N',N' - Äthylendiamin - tetraessigsäure (EDTA) dient.

4. Verwendung von mindestens einem physiologisch verträglichen paramagnetischen Komplexsalz nach Anspruch 1 zur Herstellung von Mitteln für die NMR-Diagnostik, wobei als Aminopolycarbonsäure, N,N,N',N'',N'' - Diäthylentriamin - pentaessigsäure (DTPA) dient.

**Revendications**

1. Emploi pour la préparation d'agents destinés à l'établissement de diagnostics par RMN d'un ou de plusieurs sels complexes paramagnétiques physiologiquement compatibles, d'acides amino - polycarboxyliques de formules 1 à 4 ci-dessous:

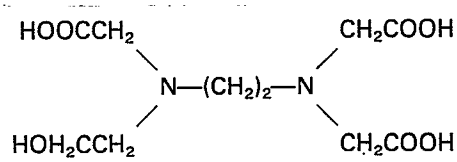

(I)

Acide N-hydroxyéthyl-N-N',N'-éthylène-diamine-triacétique (HEDTA)

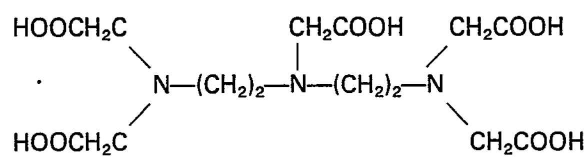

(II)

Acide N,N,N',N'',N''-diéthylène-triamine-pentaacétique (DTPA)

$$HOH_2C-CH_2N(CH_2COOH)_2 \quad (III)$$

Acide N-hydroxyéthyl-iminodiacétique

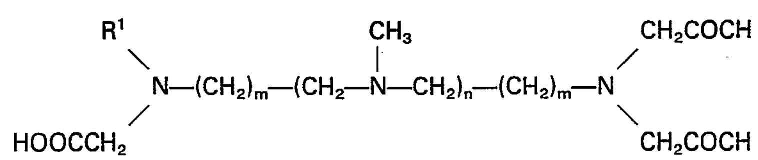

(IV),

m étant un nombre de 1 à 4,

n un nombre de 0 à 2, et

$R^1$ un radical hydrocarboné saturé ou insaturé ayant de 4 à 12 atomes de carbo ou le groupe $-CH_2-COOH$, et

des ions des lanthanides de numéros atomiques 57 à 70 ou

des ions des métaux de transition des numéros atomiques 21 à 29, 42 et 44, avec éventuellement une base minérale.

2. Emploi selon la revendication 1 des sels complexes paramagnétiques indiqués, avec de l'hydroxyde de sodium comme base minérale.

3. Emploi selon la revendication 1, des sels complexes paramagnétiques indiqués, l'acide amino-polycarboxylique étant l'acide N,N,N',N'éthylène - diaminetétracétique (EDTA).

4. Emploi selon la revendication 1 des sels complexes paramagnétiques indiqués, l'acide amino-polycarboxylique étant l'acide N,N,N',N'',N'' - diéthylènetriamine - pentaacétique (DTPA).

## Claims

1. The use of at least one physiologically tolerable paramagnetic complex salt made from aminopolycarboxylic acids having the formulae I to IV

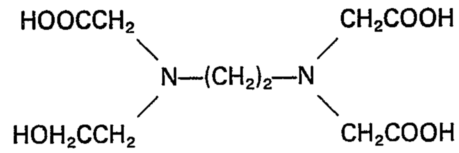

(I)

N-hydroxyethyl-N,N',N'-ethylenediaminetriacetic acid (HEDTA),

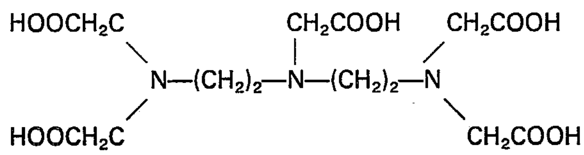

(II)

N,N,N',N'',N''-diethylenetriaminepenta-acetic acid. (DTPA),

$$HOH_2C{-}CH_2N(CH_2COOH)_2 \qquad (III)$$

N-hydroxyethylimino-diacetic acid,

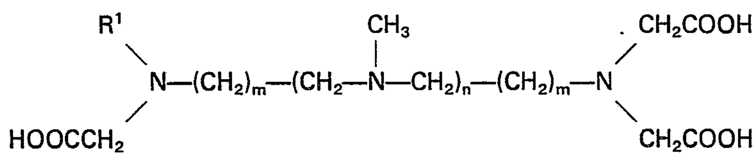

(IV)

wherein

m represents the numbers 1 to 4,

n the numbers 0 to 2,

$R^1$ a saturated or unsaturated hydrocarbon radical having from 4 to 12 hydrocarbon atoms or the group $-CH_2-COOH$, and

from the ions of the lanthanide elements having the atomic numbers 57 to 70 or from the ions of the transition metals having the atomic numbers 21 to 29, 42 and 44 and,

optionally

from an inorganic base

for the preparation of agents for NMR diagnostics.

2. The use of at least one physiologically tolerable paramagnetic complex salt according to Claim 1 for the preparation of agents for NMR diagnostics, sodium hydroxide being employed as the inorganic base.

3. The use of at least one physiologically tolerable paramagnetic complex salt according to Claim 1 for the preparation of agents for NMR diagnostics, N,N,N',N' - ethylenediaminetetra - acetic acid (EDTA) serving as the aminopolycarboxylic acid.

4. The use of at least one physiologically tolerable paramagnetic complex salt according to Claim 1 for the preparation of agents for NMR diagnostics, N,N,N',N'',N'' - diethylenetriaminepenta - acetic acid (DTPA) serving as the aminopolycarboxylic acid.